Europäisches Patentamt

European Patent Office

Office européen des brevets

(11) Publication number: **0 135 991**
**A1**

# (12) EUROPEAN PATENT APPLICATION

(21) Application number: **84304636.8**

(51) Int. Cl.⁴: **A 61 M 1/00**

(22) Date of filing: **06.07.84**

(30) Priority: **06.07.83 GB 8318325**

(43) Date of publication of application: **03.04.85**
**Bulletin 85/14**

(84) Designated Contracting States: **AT BE CH DE FR GB IT LI LU NL SE**

(71) Applicant: **Barr, Richard Henry Howard, Shaldon Road, Newton Abbot Devon, TQ12 4SQ (GB)**

(72) Inventor: **Barr, Richard Henry Howard, Shaldon Road, Newton Abbot Devon, TQ12 4SQ (GB)**

(74) Representative: **Robinson, Anthony John Metcalf et al, Kilburn & Strode 30 John Street, London, WC1N 2DD (GB)**

(54) Surgically implantable hydrocephalus valve.

(57) A surgically implantable hydrocephalus valve comprising a valve body (1) containing a ball valve (5, 6) loaded by a spring (12) whose prestress can be set to a fixed but adjustable position by rotation by a screwdriver of a cog wheel (21) engaging a rack (18) on the spring abutment (14).

EP 0 135 991 A1

1.

## SURGICALLY IMPLANTABLE HYDROCEPHALUS VALVE

This invention relates to surgically implantable hydrocephalus valves. Such valves are used in a ducting system or shunt used for draining cerebrospinal fluid (CSF) from a cerebral ventrical to another part of the body such as the peritoneal cavity or the jugular vein.

A hydrocephalus valve is in the form of a loaded check valve so that it both prevents return flow while permitting flow away from the brain and also maintains a certain pressure in the ventricle depending upon the value of the bias. While it would be possible to set the bias at a level such that pressure in the ventricle is maintained at a low level it is thought that this would deal with one of the symptoms of the illness without encouraging the body to correct the fault so that there would be little tendency towards a long-term cure. In GB-A-1 434 107 Salomon Hakim describes a shunt system in which the bias on the hydrocephalus valve is under continuous adjustment by means of a control system incorporating a sensor located in the dural region between the brain and the skull to sense the pressure exerted within the cranial cavity. As the sensed pressure increases, the bias on the valve is correspondingly reduced. Thus, while this system provides for variation in the bias on the valve the relationship between the pressure in the cranial cavity and the bias cannot be adjusted once the apparatus is implanted. Moreover the shunt system is complex and its use involves additional surgical proceedures.

In EP-A1-0 060 369 there is described an implantable hydrocephalus valve comprising a cylindrical chamber having an inlet forming a concial seat containing a valve closure member in the form of a ball and a diametrically opposite outlet. A bar of magnetic material is mounted within the chamber for rotation about its mid-point, the axis of rotation coinciding with the axis of the cylinder. One end of the bar carries a cantilevered arcuate leaf spring which engages the valve ball and provides a spring bias on the ball of a strength which depends on the angular position of the bar. The valve, when implanted, can be adjusted with the aid of a magnet which acts through the wall of the chamber and the skin covering it. A problem presented by this valve is that it is extremely difficult, if not impossible, to position the bar accurately and with certainty. In FR-A1-2 354 103 there is described another implantable hydrocephalus valve operated from outside the body by means of an external magnet acting on an internal magnet. This valve uses an inductive receiver to detect movements of a nut whose rotation by rotation of the internal magnet attached thereto is used to adjust the valve. This construction requires a complex and delicate valve operating system; since reliability of a hydrocephalus valve is vital, simplicity and sturdiness are most desirable. Moreover, the presence of a transmitter system and the complexity of the valve operating mechanism lead to bulk; since a hydrocephalus valve has to be implanted between the skin and the skull, minimum dimensions are desirable. A further hydrocephalus valve is described in GB-A-1 301 124 in which provision is made for adjusting the bias applied by a coil spring to a valve closure member but such adjustment can only be made prior to implantation and there is no provision for subseqent adjustment.

It has now been perceived that there is a need for a small, simple, robust and reliable hydrocephalus valve which can be preset to open at a required pressure differential and which has means by which the said differential setting can be adjusted from time to time after implantation in a manner which is simple and reliable, that is to say the surgeon can know with certainty that the valve has been reset to operate at the newly required differential.

The need explained above is met by the invention according to which there is provided a surgically implantable hydrocephalus valve comprising a valve body, a valve seat, a valve closure element, a spring biassing the closure element towards the seat, and an adjustment element which engages the spring and whose position relative to the valve body determines the spring load effective on the closure element, characterised by mechanical setting means for setting the adjustment element to selected positions, the setting means comprising a setting element physically accessible from immediately outside the valve body and transmission means extending through the valve body to the interior thereof to transmit movements of the setting element to the adjustment element.

Thus, the hydrocephalus valve is not under continuous control but has means whereby the bias load can be adjusted from time to time. Thus, in use the surgeon initially selects the adjustment required to give the valve bias that he considers appropriate to the patient and implants shunt system including the hydrocephalus valve. After a period of time which may be determined by the reactions of the patient or by appropriate tests and which may amount to several months, the surgeon may decide that the patient has progressed sufficiently for a higher bias load to be appropriate and he will uncover the setting element and adjust the setting of the valve. Since the setting element is directly accessible to the surgeon he can adjust the setting element precisely to the position required and since the setting element is connected to the adjustment element by transmission means, the surgeon can be certain that by positioning the setting element to the required position he has positioned the adjustment element to the required position. In other words, each position of the setting element corresponds to a particular position of the adjustment element and thus to a particular differential pressure at which the valve will open. The setting means can be so constructed that only a very small incision is necessary to obtain access to it for adjustment and such access can be obtained a number of times without difficulty.

In a preferred arrangement the adjustment element carries rack teeth and the setting means includes a cog wheel engaging the rack and carried on a shaft extending out of the valve body. The outer end of the shaft may be formed with means such as a screwdriver slot for engagement by a rotation tool. Preferably the adjustment element is generally tubular and receives part of the length of the compression coil spring therein.

5.

In a preferred construction, the valve closure member comprises a ball held adjacent the valve seat by a cage.

The invention may be carried into practice in various ways but one hydrocephalus valve embodying the invention will now be described by way of example with reference to the accompanying drawings, in which:

Figure 1 is a longitudinal section of the valve;

Figure 2 is a cross section on the line II-II in Figure 1; and

Figure 3 is a cross section on the line III-III in Figure 1.

The general construction and mode of use of a hydrocephalus shunt is well known and will not be described in detail. It is usual in such a shunt to employ two valves in series joined by a flexible duct whose purpose is both as an inspection point at which it can be ascertained that flow is occurring through the shunt and as a pumping chamber to induce flow through the shunt to clear any blockages that may occur. The valve to be described may be used as only one of the valves of the pair, the other valve being a simple spring-loaded check valve. Alternatively both valves could be of the construction to be described.

The valve shown in the drawings comprises a valve body 1 comprising a generally tubular portion 2, an inlet end fitting 3 and an outlet end fitting 4. Each of the end fittings has a spigot only part of which is shown and to which is secured a flexible tube affording part of the complete hydrocephalus shunt system. The inlet end fitting 3 has a conical valve seat 5 with which a polished sapphire ball 6 cooperates to provide the sealing action, the ball constituting a valve closure element. The ball can move away from the seat 5 to open the valve by a distance which is limited by a cage 7 in the form of a cup which is secured to a rebate 8 on the end fitting 3 and has a central opening 9. The rim of the opening 9 is formed with three semi-circular notches 11 to allow flow past the ball when it is engaged with the rim of the opening 9. The ball 6 is biassed towards engagement with the valve seat 5 by a compression coil spring 12 which is of fine, close coiled, shallow pitch construction and engages, at the end remote from the ball 6, against a shoulder 13 towards the remote end of the bore of a sleeve-shaped adjustment element or abutment member 14. The internal diameter of the abutment member 14 is slightly greater than the outside diameter of the spring 12 so that it is able to afford lateral support to the spring without restricting its movement. The shoulder 13 surrounds an opening 15 leading to the bore 16 in the outlet end fitting 4. On one side, the abutment member 14 is formed with teeth 17 forming a rack 18, the bases of the teeth forming chords across the abutment member 14 and the crests being constituted by continuations of the cylindrical outer surface of the abutment member.

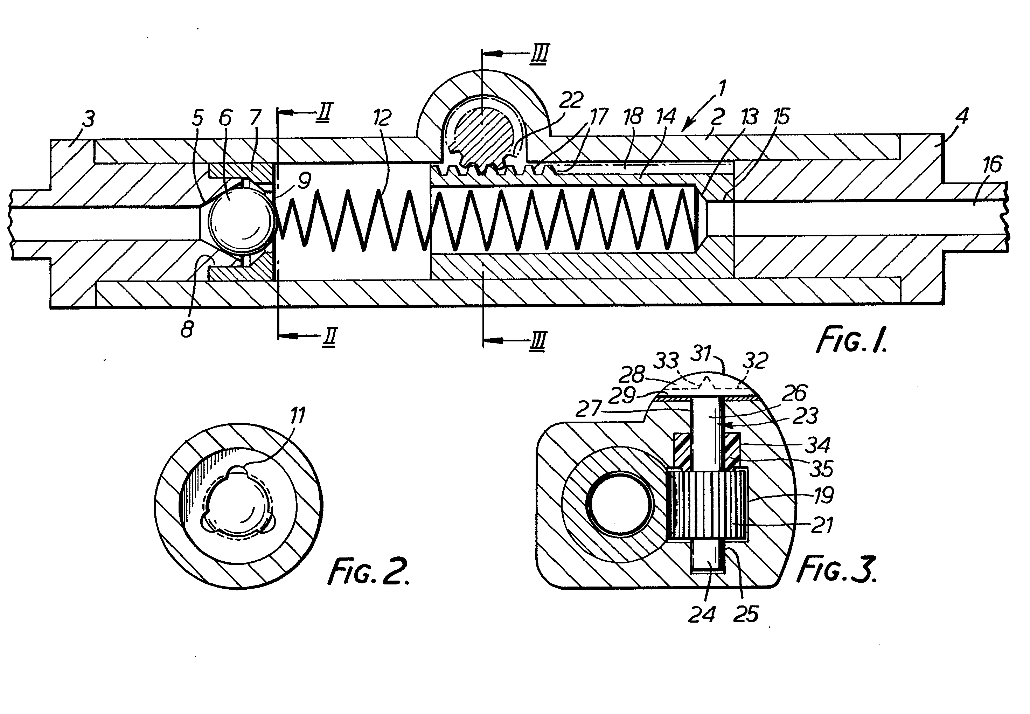

The generally cylindrical shape of the valve body 1 is modified in the region of the cross section of Figure 3 to provide a chamber 19 in which rotates a cog wheel 21 whose teeth 22 mesh with the teeth 17 of the rack 18. The cog wheel 21 is integral with a shaft 23 having a stub 24 mounted in a counterbore 25 in the housing and a main portion 26 which extends through a bore 27 in the housing to a head 28 having a flat end surface 29 lying against a corresponding flat surface on the housing and having a rounded outer surface 31 which forms a continuation of the adjacent surfaces of the housing so that the housing and head together present a generally smooth outer surface. Extending across the head 28 is a screwdriver slot 32 with a central transverse ridge 33 so that the cog wheel 21 may be turned by a screwdriver blade having a notch therein to cooperate with the ridge 33 to prevent the blade slipping in the slot 32. The chamber 19 includes a portion 34 of reduced diameter containing a friction bush 35.

All parts of the valve with the exception of the friction bush 35 are made of stainless steel or biocompatible plastics material while the friction bush 35 is also made of a material which can be subjected to elevated temperatures or irradiation to enable the whole valve to be sterilised.

Operation

The valve shown in the drawings forms part of a hydrocephalus shunt as described. Prior to its implantation, the surgeon decides the prestress to be applied to the ball 6 and rotates the head 28 constituting setting means until the slot 32 achieves the angular position relative to the valve body 1 which will via transmission means constituted by the shaft 23 and the cog wheel 21 position the abutment member to compress the spring 12 by the required amount. Because the valve is manufactured with precision, each rotational position will closely correspond to a particular spring load which, in turn, corresponds to

8.

a level of pressure drop across the valve at which the valve opens.

The shunt is then implanted in the patient with the valve located so that after the incision has healed the head 28 will be located just below the skin of the patient. When the surgeon decides that a change in the setting of the valve is required, he makes a small incision adjacent the slot 32 and introduces a screwdriver to turn the shaft 23 the required amount. Such re-setting can be accomplished from time to time as necessary.

0135991

CLAIMS

1.  A surgically implantable hydrocephalus valve
comprising a valve body (1), a valve seat (5), a
valve closure element (6), a spring (12) biassing
the closure element towards the seat, and an adjustment element (14) which engages the spring (12) and
whose position relative to the valve body (1) determines the spring load effective on the closure
element, characterised by mechanical setting means
for setting the adjustment element to selected positions,
the setting means comprising a setting element (28)
physically accessible from immediately outside the
valve body and transmission means (23) extending
through the valve body to the interior thereof to
transmit movements of the setting element (28) to the
adjustment element (14).

2.  A surgically implantable hydrocephalus valve
according to Claim 1 in which the valve body (1) is
generally tubular, the valve closure element (6) is
movable generally axially of the valve body, the
adjustment element (14) is movable generally axially
of the valve body, and the spring (12) is a compression
coil spring extending generally axially of the valve
body between the valve closure element and the adjustment element.

3.  A surgically implantable hydrocephalus valve
according to Claim 2 wherein the adjustment element (14)
carries rack teeth (17) and the setting means includes
a cog wheel (21) engaging the rack.

4.    A surgically implantable hydrocephalus valve according to Claim 3 wherein said transmission means comprises a shaft (23) on which said cog wheel (21) is mounted, said shaft extending out of the valve body.

5.    A surgically implantable hydrocephalus valve according to Claim 4 wherein the outer end of the shaft constitutes said setting element (28), said outer end being formed with means (32) for engagement with a rotation tool.

6.    A surgically implantable hydrocephalus valve according to any of Claims 2 to 5 wherein the adjustment element (14) is generally tubular and receives part of the length of the compression coil spring (12) therein.

7.    A hydrocephalus valve shunt comprising a cerebroventricular catheter, a discharge catheter, and a duct system connecting the two catheters and including therein a surgically implantable hydrocephalus valve as claimed in any of Claims 1 to 6.

8.    A hydrocephalus valve shunt according to Claim 7 which includes a second check valve in series with the said surgically implantable hydrocephalus valve.

0135991

FIG. 1.

FIG. 2.

FIG. 3.

European Patent
Office

**EUROPEAN SEARCH REPORT**

. Application number

| DOCUMENTS CONSIDERED TO BE RELEVANT | | | EP 84304636.8 |
|---|---|---|---|

| Category | Citation of document with indication, where appropriate, of relevant passages | Relevant to claim | CLASSIFICATION OF THE APPLICATION (Int. Cl.4) |
|---|---|---|---|
| Y | GB - A - 1 500 458 (CORDIS CORP.)<br>  * Totality * | 1,2,6-8 | A 61 M 1/00 |
| Y | US - A - 4 387 715 (S. HAKIM)<br>  * Fig. 8-15; column 5, line 8 - column 6, line 49 * | 1,2,6-8 | |
| A | GB - A - 1 553 485 (MESSERSCHMITT B.)<br>  * Fig. 1,2; page 2, lines 68-103 * | 1,7 | |
| D | & FR-A-2 354 103 | | |
| A | CATALOGUE, FA. CORDIS; Erkrath, BRD, November 1982, "Neuro-chirurgische Produkte" pages 1-4<br>  * Pages 1-4 * | 1,2,6-8 | **TECHNICAL FIELDS SEARCHED (Int. Cl.4)**<br><br>A 61 M 1/00 |

| The present search report has been drawn up for all claims | | |
|---|---|---|
| Place of search<br>VIENNA | Date of completion of the search<br>23-11-1984 | Examiner<br>LUDWIG |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO Form 1503. 03 82